(19) **Europäisches Patentamt / European Patent Office / Office européen des brevets**

(11) **EP 4 485 467 A1**

(12) # EUROPEAN PATENT APPLICATION

(43) Date of publication:
**01.01.2025 Bulletin 2025/01**

(21) Application number: **23461610.0**

(22) Date of filing: **27.06.2023**

(51) International Patent Classification (IPC):
***G16C 60/00*** (2019.01)

(52) Cooperative Patent Classification (CPC):
**G16C 60/00;** G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **QSAR LAB SPOLKA Z OGRANICZONA ODPOWIEDZIALNOSCIA**
**80-172 Gdansk (PL)**

(72) Inventors:
• **WYRZYKOWSKA, Ewelina**
**19-300 ELK (PL)**

• **STEPNIK, Maciej**
**94-126 LODZ (PL)**
• **WOJCIECHOWSKA, Alicja**
**06-300 PRZASNYSZ (PL)**
• **NIMZ, Kinga**
**83-200 STAROGARD GDANSKI (PL)**
• **GROMELSKI, Maciej**
**82-300 ELBLAG (PL)**
• **PUZYN, Tomasz**
**80-438 GDANSK (PL)**

(74) Representative: **AOMB Polska Sp. z.o.o.**
**ul. Rondo Ignacego Daszynskiego 1**
**00-843 Warsaw (PL)**

(54) **COMPUTATIONAL METHODS FOR PREDICTION OF SILICA DIOXIDE NANOPARTICLES' MUTAGENIC AND GENOTOXIC EFFECTS TO HUMAN HEALTH**

(57) The invention relates to two computational methods for prediction of mutagenic or genotoxic effects to human health induced by silica dioxide ($SiO_2$) nanoparticles. The methods are based on Qualitative Structure-Activity Relationship (QSAR) modelling using an original combination of statistical and machine learning methods i.e., Logistic Principal Component Analysis (L-PCA) and Support Vector Machines Classifier (method A) or Decision Tree Classifier (method B). The method A responds to the estimation of mutagenic response that can be used as an equivalent of experimental measurement under OECD 487 protocol i.e., *in vitro* micronucleus assay on mammalian cell lines. The method B responds to the estimation of genotoxic response that can be used as an equivalent of experimental measurements with *in vitro* comet assay on mammalian cell lines. Methods A and B utilize as an input the information about the characteristic of nanoparticles and biological model of interest i.e., a type of cell line. The outcome from the prediction classifies the investigated silica dioxide nanoparticles as giving positive or negative response for the defined biological model in a proper test assay (micronucleus assay - method A, comet assay - method B).

Fig. 1.

## Description

### Technical Field

The invention relates to computational methods for prediction of silica dioxide nanoparticles mutagenic and genotoxic effects to human health, based on Qualitative Structure-Activity Relationship (QSAR) modelling using an original combination of statistical and machine learning methods i.e., Logistic Principal Component Analysis (L-PCA) and Support Vector Machines Classifier (method A) or Decision Tree Classifier (method B). The method A responds to the estimation of mutagenic response that can be used as an equivalent of experimental measurement under OECD 487 protocol i.e., in vitro micronucleus assay on mammalian cell lines. The method B responds to the estimation of genotoxic response that can be used as an equivalent of experimental measurements with in vitro comet assay on mammalian cell lines.

### Background

Silica nanoparticles ($SiO_2$ NPs) have been widely used in various fields, such as biomedicine, electronics, and cosmetics due to their unique physicochemical properties. However, their potential genotoxicity and mutagenicity have raised concerns about their safety. The European Commission Regulation (EU, 2018/1881) has classified $SiO_2$ NPs as a Category 2 mutagenic substance, meaning that they have the potential to cause genetic damage. Additionally, other regulatory bodies such as the US Environmental Protection Agency (EPA) and the International Conference on Harmonization of Technical Requirements for Registration of Pharmaceuticals for Human Use (ICH) have also established guidelines for the assessment of genotoxicity and mutagenicity of silica nanoparticles.

The guidelines emphasize the importance of conducting genotoxicity studies using validated and internationally recognized in vitro methods, such as the Ames test, the micronucleus assay, and the comet assay. The micronucleus assay is a cytogenic assay that detects the presence of micronuclei, which are small nuclei formed from chromosomal fragments that were not incorporated into the main nucleus during cell division. The comet assay is a sensitive and versatile technique that can detect DNA damage and repair in individual cells. The in vitro assays typically involve the use of cultured cells, such as bacterial or mammalian cells, and assess endpoints such as DNA damage, chromosomal aberrations, and gene mutations. On the other hand, in vivo assays involve the use of animals and assess endpoints such as micronucleus formation, DNA adducts, and gene mutations. Regulatory bodies also specify the minimum requirements for conducting genotoxicity studies. For example, the ICH guideline for genotoxicity testing recommends the use of at least two independent assays to confirm the results and the inclusion of both positive and negative controls to ensure the reliability of the data. In addition to these regulatory guidelines, ethical aspects must also be considered when conducting genotoxicity studies of $SiO_2$ NPs.

The Regulation (EC) No. 1907/2006 of the European Parliament and the Council from December 18, 2006, on the Registration, Evaluation, Authorization and Restriction of Chemicals (REACH) emphasizes the necessity of detailed chemical risk assessment of any chemical substances introduced on the European market. However, the use of animals in in vivo studies raises ethical concerns, and alternative methods, such as in vitro assays using human cells or computational modelling, should be considered whenever possible to fulfil the 3Rs' principles - Replacement, Reduction and Refinement of animal testing - proposed by Russell and Burch (The Principles of Humane Experimental Technique, 1959). One of the proposed groups of alternative methods is computational approach like Quantitative/Qualitative Structure-Activity Relationship (QSAR) modelling.

QSAR is a method used to establish a relationship between chemical structures and observed biological activity. In QSAR modelling, the structure of chemical substances is described in a numerical manner with so-called descriptors, which are used as input variables for regression and classification models. Regression models are used to predict continuous values of endpoints e.g. EC50, while classification models are used to predict categorical response of endpoints such as positive or negative genotoxic effect. The relationship between chemical structure expressed with descriptors and observed activity, is identified through applying machine learning algorithms like multiple linear regression (MLR), support vector machines (SVM), decision tree, random forests, XGBoost, AdaBoost, etc. QSAR modelling offers the benefit of identifying the fundamental mechanisms that govern the structural characteristics of substances, as well as the specific pathways that result in adverse effects. More importantly, QSAR models allow for the prediction of unknown activity for newly considered chemicals, even not synthesized yet, based solely on the descriptors included in the model, without carrying out time-consuming and costly experimental testing.

From the state of the art, a patent solution No. WO16201789A1 is known, disclosing QSAR toxicity prediction method to evaluate health effects of nano-metal oxides in particular. Specifically, the method comprises: predicting a toxicity endpoint of unknown metallic oxide according to a quantitative relationship between structural features and cytotoxicity effect of the nano-crystalline metal oxide; and is a method of building a nano-crystalline metal oxide prediction model by combining a physicochemical structure parameter and a special mechanism of toxication of the nano-crystalline metal oxide, and applying the nano-crystalline metal oxide prediction model to predict the unknown toxicity endpoint.

**Summary**

The main objective of the invention is to develop dependable and effective methods for prediction of silica dioxide nanoparticles mutagenic and genotoxic effects to human health, offering a cost-effective and ethical solution in comparison to experimental testing. The invention described in the patent application provides a way to predict mutagenicity - method A, and genotoxicity - method B of $SiO_2$ nanoforms.

According to the first aspect of the present invention, this object is achieved by a computational method for prediction of silica dioxide nanoparticles mutagenic effects to human health, based on QSAR modelling. The method characterized by including providing datasets of $SiO_2$ nanoforms and cell line characteristics with corresponding positive/negative responses observed in micronucleus assay, and used as a training set and a validation set;

reduction of the multidimensionality of cell line characteristic dataset to two first principal components, PC1 and PC2, explaining more than 70% of deviation using L-PCA, pre-processing of PC1, PC2 and minimal particle size of $SiO_2$ through standardization, and reconstruction of the Support Vector Machines Classifier with inputs for the training set matrices of PC1 and PC2 describing cell lines and vector of $SiO_2$ nanoforms size, and vector of corresponding biological responses in micronucleus assay;

a new samples mapping into PC1 and PC2 retaining all parameters from the L-PCA algorithm carried out for the training set, where the new samples are described through a value of nanoforms' minimal size within the indicated range, and a cell line characteristic through indicated adherence to the particular group with the binary scale, where the characteristics should be as follows:

Minimal size - minimum size of nanoparticle in the sample, quantitative factor, derived from TEM images: 5 - 224.8 [nm]

Cell line origin:
human [1 or 0]

Cell type and organ of origin:

epithelium lung [1 or 0]

fibroblast adipose tissue [1 or 0]

mononuclear cell peripheral blood (lymphocytes, monocytes)

epithelium colon

endothelium umbilical vein

Germ layer the cells originate from:
endoderm [1 or 0]

Biological nature of cell used:
cancer [1 or 0], and

application of the developed QSAR model for a newly defined samples and prediction as positive or negative based on provided descriptors.

Preferably, the method using the following steps: datasets preparation, datasets splitting into a training set and a validation set, L-PCA for the training set, standardization for the training set, model calibration for the training set, internal validation for the training set, validation set mapping, validation set scaling, prediction for the validation set, external validation, new samples defining, new samples mapping, new samples scaling, predicting mutagenic effects for the new sample.

According to the second aspect of the present invention, this object is achieved by a computational method for prediction of silica dioxide nanoparticles genotoxic effects to human health, based on QSAR modelling. The method

**EP 4 485 467 A1**

characterized by including providing datasets of $SiO_2$ nanoforms and cell line characteristics with corresponding positive/negative responses observed in comet assay, and used as a training set and a validation set;

reduction of the multidimensionality of cell line characteristic dataset to two first principal components, PC1 and PC2, explaining more than 70% of deviation using L-PCA, pre-processing of PC1, PC2 and minimal particle size of $SiO_2$ through standardization, and reconstruction of the Decision Tree Classifier with inputs for the training set matrices of PC1 and PC2 describing cell lines and vector of $SiO_2$ nanoforms size, and vector of corresponding biological responses in comet assay;

mapping of new samples into PC1 and PC2 retaining all parameters from the L-PCA algorithm carried out for the training set, where the new samples are described through a value of nanoforms' minimal size within the indicated range, and a cell line characteristic through indicated adherence to the particular group with the binary scale (1 - yes, 0 - no), where the characteristics should be as follows:

Minimal size - minimum size of nanoparticle in the sample, quantitative factor, derived from TEM images: 5 - 166.1 [nm]

Cell line origin:

human [1 or 0]

mouse [1 or 0]

hamster [1 or 0]

rat [1 or 0]

Cell type and organ of origin:

fibroblast embryo [1 or 0]

epithelium colon [1 or 0]

epithelium lung [1 or 0]

neuroblasts brain [1 or 0]

keratinocytes skin [1 or 0]

mononuclear cell peripheral blood (lymphocytes, monocytes) [1 or 0]

endothelium umbilical vein [1 or 0]

lymphoblast spleen [1 or 0]

epithelium ovary [1 or 0]

fibroblast lung [1 or 0]

macrophage monocytes [1 or 0]

epithelial kidney [1 or 0]

embryo kidney [1 or 0]

Germ layer the cells originate from:

ectoderm [1 or 0]

mesoderm [1 or 0]

endoderm [1 or 0]

Biological nature of cell used:
cancer [1 or 0], and

application of the developed QSAR model for the newly defined samples and prediction as positive or negative based on provided descriptors.

Preferably, the method using the following steps: datasets preparation, datasets splitting into a training set and a validation set, L-PCA for the training set, standardization for the training set, model calibration for the training set, internal validation for the training set, validation set mapping, validation set scaling, prediction for the validation set, external validation, new samples defining, new samples mapping, new samples scaling, predicting genotoxic effects for the new sample.

The provided QSAR models use data extracted from open literature on mutagenicity and genotoxicity induction in mammalian cells caused by exposure to various $SiO_2$ nanoforms, investigated by micronucleus and comet assays. Additional information on each *in vitro* experiment, such as cell line origin (e.g., human, mouse, rat), cell type and organ of origin (e.g., epithelium from colon, fibroblast from lung, neuroblasts from brain), germ layers (e.g., endoderm, ectoderm, and mesoderm), and biological nature of the cells used (normal, cancer), is employed to extend the set of nanoform descriptors. By incorporating these supplementary descriptors, the presented approaches can identify the specific sensitivity of exposed cell lines to $SiO_2$ nanoforms in inducing mutagenicity and genotoxicity effects. Thus, the predictions of the QSAR models are tailored to the particular cell lines investigated by the user. Furthermore, unlike the traditional QSAR modelling approach, where model calibration is limited by the number of samples in the training set (1 descriptor in the model for at least 5 chemicals in the training set), the presented approaches utilize dimensionality reduction of the original datasets through Logistic Principal Component Analysis (L-PCA). L-PCA algorithm allows for analyzing a multidimensional, binary scale data, where information from the original matrices is condensed in new variables, so called principal components (PCs), and the total amount of explained information is expressed as deviation percentage. The PCs are mutually orthogonal and explain unique portion of the information, with the first PC retaining the most information and subsequent PCs retaining less. In the presented methods A and B, PCs cover the cell line information in at least 70% of deviation, and to meet the 1:5 rule, the first two PCs are qualified for QSAR modelling. Obtained PCs for cell line characteristics and original values of minimum particle size of $SiO_2$ are then standardized and used as input variables in QSAR modelling using an indicated machine learning algorithm with specified hiperparameters.

Each QSAR model is developed and validated using open experimental data for a set of $SiO_2$ nanoparticles obtained with recommended in vitro micronucleus assay (method A) or comet assay (method B). The relationships between cell lines and the minimal nanoparticles size with the observed mutagenic and genotoxic activity, allow the QSAR models to predict the mutagenicity and genotoxicity for new, untested $SiO_2$ nanoparticles, eliminating the need for experimental testing.

**Drawing**

The invention may be performed in various ways, and, by way of example only, embodiments thereof will now be described, reference being made to the accompanying drawing:

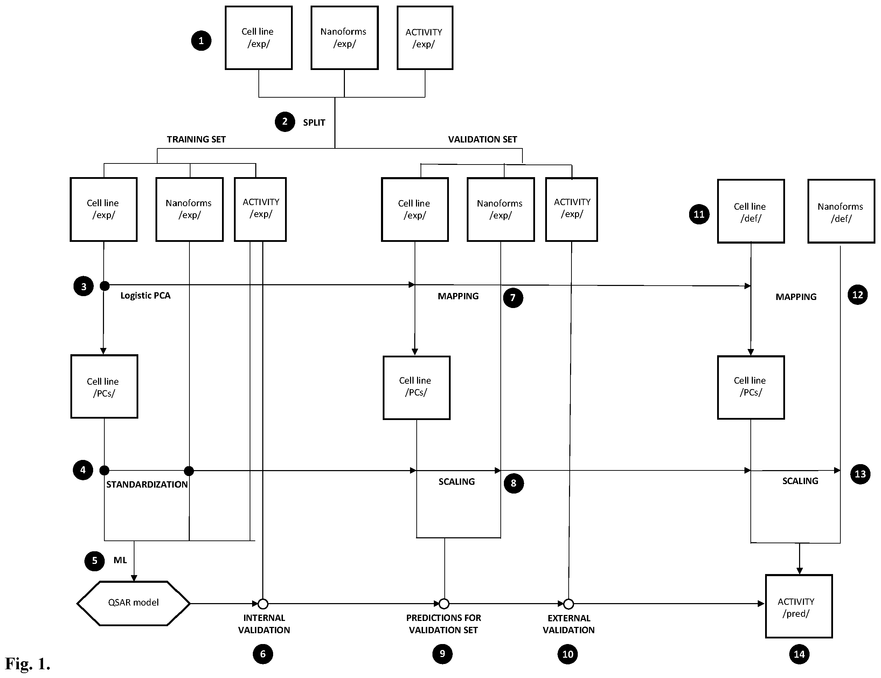

Figure 1 - Scheme of steps required in execution of method A and method B provided in the invention.

**Detailed description**

The invention comprises of two methods for predicting mutagenic (method A) and genotoxic effects (method B) induced by $SiO_2$ nanoparticles, being an equivalent of *in vitro* testing with micronucleus and comet assay, respectively. Method A and method B enable the prediction of positive/negative response in mammalian cell lines according to the general scheme presented in Figure 1, where:

ACTIVITY - positive/negative response in Micronucleus assay (method A) or Comet assay (method B);

Cell line - matrix of cell line characterization;

Nanoforms - matrix of nanoforms characterization;

Logistic PCA - logistic principal component analysis (L-PCA);

ML - machine learning algorithm;

/exp/ - experimental data;

/def/ - nanoforms and cell lines defined by user;

/PCs/ - principal components;

/pred/ - predicted response;

> • fitting and transformation of the training set, which additionally calculates and provides parameters to enable transformation of validation/new dataset
> ◦ Predictions/comparison with experimental data and calculation of statistical parameters, with no influence on other datasets.

To implement the invention, both methods require to recreate QSAR models according to the guidance provided by the Authors in this document. For method A and B, there is obligatory to provide *in vitro* experimental dataset used for a proper QSAR model development, L-PCA analysis, pre-processing of selected principal components and minimal particle size of $SiO_2$, and reconstruction of the QSAR model using a defined machine learning algorithm with input parameters applied for the training set. The obtained statistical parameters for training and validation sets converged with these presented in this document, confirm the correct reconstruction of a proper QSAR model and its willingness for new predictions. Subsequently, new samples have to be mapped (transformed) into L-PCA performed for the training set, to generate inputs for the QSAR model and execution of predictions for new samples. The applicability domain for each method is limited to the range of descriptor values for nanoforms and tested cell lines.

According to the invention (methods A and B), prediction of mutagenic and genotoxic potential of new, untested $SiO_2$ nanoparticles, requires information about the nanoform's structure characteristics (such as minimum particle size), cell line characteristics; and QSAR models reconstructed according to the provided guidance. The credibility of the predictions is confirmed with the applicability domain of the applied model through assessing the convergence of the characteristic of newly defined sample with the samples in the training set of a proper model.

**METHOD A - QSAR model for prediction mutagenicity of $SiO_2$ nanoparticles expressed with *in vitro* micronucleus assay.**

Method A employs a battery of common data curation and machine learning algorithms: dataset splitting based on random split algorithm, dimensionality reduction based on logistic principal component analysis, standardization, and model development based on support vector machines classifier. Said algorithms can be executed by means of any programming language and/or data analysis software.

The general scheme to recreate method A of the invention is presented in Figure 1. The procedure is divided into 14 steps described below:

STEP 1. Datasets preparation 1

Method A is based on an *in vitro* experimental data available for limited number of samples described with: i) cell lines and nanoforms characterization (Table A_1), and ii) observed mutagenic response (Table A_2). The observed responses for the available samples are as follows:

[positive response] 10 samples
[negative response] 18 samples

*Table A_1.     Cell line characteristics and SiO$_2$ nanoform characteristic of samples used in development and validation of method A (T – training set, V – validation set)*

| ID | Cell line | Split | Cell line characteristic | | | | | | | | Nanoform characteristic |
|----|-----------|-------|--------|-----------------|--------------------------|-----------------------------|-------------------|------------------------------|--------|----------|-------------------------|
| | | | Human | Epithelium lung | Fibroblast adipose tissue | Mononuclear cell peripheral | Epithelium colon | Endothelium umbilical vein | Cancer | Endoderm | Minimum particle size |
| 0 | A549 | T | 1 | 1 | 0 | 0 | 0 | 0 | 1 | 1 | 13.9 |
| 1 | A549 | T | 1 | 1 | 0 | 0 | 0 | 0 | 1 | 1 | 52.1 |
| 2 | A549 | T | 1 | 1 | 0 | 0 | 0 | 0 | 1 | 1 | 94.1 |
| 3 | 3T3-L1 | T | 0 | 0 | 1 | 0 | 0 | 0 | 0 | 0 | 8.8 |
| 4 | 3T3-L1 | V | 0 | 0 | 1 | 0 | 0 | 0 | 0 | 0 | 29.7 |
| 5 | 3T3-L1 | T | 0 | 0 | 1 | 0 | 0 | 0 | 0 | 0 | 27.6 |
| 6 | 3T3-L1 | T | 0 | 0 | 1 | 0 | 0 | 0 | 0 | 0 | 224.8 |
| 7 | PBL | V | 1 | 0 | 0 | 1 | 0 | 0 | 0 | 0 | 15 |
| 8 | PBL | V | 1 | 0 | 0 | 1 | 0 | 0 | 0 | 0 | 55 |
| 9 | A549 | T | 1 | 1 | 0 | 0 | 0 | 0 | 1 | 1 | 50 |
| 10 | A549 | T | 1 | 1 | 0 | 0 | 0 | 0 | 1 | 1 | 200 |
| 11 | Caco-2 | V | 1 | 0 | 0 | 0 | 1 | 0 | 1 | 1 | 51.8 |
| 12 | Caco-2 | V | 1 | 0 | 0 | 0 | 1 | 0 | 1 | 1 | 14.9 |
| 13 | FE1 | T | 0 | 1 | 0 | 0 | 0 | 0 | 0 | 1 | 12 |
| 14 | FE1 | T | 0 | 1 | 0 | 0 | 0 | 0 | 0 | 1 | 5 |
| 15 | FE1 | V | 0 | 1 | 0 | 0 | 0 | 0 | 0 | 1 | 10 |
| 16 | BEAS-2B | T | 1 | 1 | 0 | 0 | 0 | 0 | 0 | 1 | 10 |
| 17 | Huvec | T | 1 | 0 | 0 | 0 | 0 | 1 | 0 | 0 | 83.8 |
| 18 | Huvec | V | 1 | 0 | 0 | 0 | 0 | 1 | 0 | 0 | 41.3 |
| 19 | Huvec | T | 1 | 0 | 0 | 0 | 0 | 1 | 0 | 0 | 20.7 |
| 20 | Huvec | T | 1 | 0 | 0 | 0 | 0 | 1 | 0 | 0 | 8.5 |
| 21 | BEAS-2B | T | 1 | 1 | 0 | 0 | 0 | 0 | 0 | 1 | 7 |
| 22 | BEAS-2B | V | 1 | 1 | 0 | 0 | 0 | 0 | 0 | 1 | 7 |
| 23 | PBL | T | 1 | 0 | 0 | 1 | 0 | 0 | 0 | 0 | 10 |
| 24 | PBL | V | 1 | 0 | 0 | 1 | 0 | 0 | 0 | 0 | 100 |
| 25 | PBL | T | 1 | 0 | 0 | 1 | 0 | 0 | 0 | 0 | 6 |
| 26 | PBL | T | 1 | 0 | 0 | 1 | 0 | 0 | 0 | 0 | 6 |
| 27 | PBL | T | 1 | 0 | 0 | 1 | 0 | 0 | 0 | 0 | 6 |

Table A_2. Mutagenic response of samples used in development and validation of method A (1 - positive, 0 - negative response) (T - training set, V - validation set)

| ID | Split | Mutagenic effect |
|----|-------|------------------|
| 0 | T | 0 |
| 1 | T | 0 |
| 2 | T | 0 |
| 3 | T | 0 |

(continued)

| ID | Split | Mutagenic effect |
|----|-------|------------------|
| 4 | V | 0 |
| 5 | T | 1 |
| 6 | T | 0 |
| 7 | V | 0 |
| 8 | V | 0 |
| 9 | T | 0 |
| 10 | T | 0 |
| 11 | V | 0 |
| 12 | V | 1 |
| 13 | T | 1 |
| 14 | T | 1 |
| 15 | V | 1 |
| 16 | T | 1 |
| 17 | T | 1 |
| 18 | V | 1 |
| 19 | T | 1 |
| 20 | T | 1 |
| 21 | T | 0 |
| 22 | V | 0 |
| 23 | T | 0 |
| 24 | V | 0 |
| 25 | T | 0 |
| 26 | T | 0 |
| 27 | T | 0 |

STEP 2. Datasets splitting 2 into a training set and a validation set

The recreation of the A model requires the same split into training and validation sets as it is available in Table A_1. The decisive split was achieved through random split algorithm (test_size=0.3, random_state=42, stratify=y). The training set is used for calibration and verification of the model robustness (internal validation). The validation set is used for verification of the model prediction ability (external validation). The ratio between number of samples in the training and validation set is 7:3 (19 training set: 9 validation set).

The number of samples with positive/negative responses in the training set is as follows:

[positive response] 7 samples
[negative response] 12 samples

The number of samples with positive/negative responses in the validation set is as follows:

[positive response] 3 samples
[negative response] 6 samples

STEP 3. L-PCA 3 for the training set

In logistic principal component analysis (L-PCA), the multidimensionality of the matrix of cell line characteristic is reduced to two first principal components (PCs: PC1, PC2), which explain 87% of the overall deviance.

The hyperparameters for L-PCA are as follows:
k=2, m=5, quiet=TRUE, partial_decomp=FALSE, max_iters=1000, conv_criteria=1e-5, random_start=FALSE, main_effects=TRUE

Obtained scores values for samples in training set are reported in the Table A_3.

*Table A_3. Scores values of samples used in development and validation of method A- (T - training set, V - validation set)*

| ID | Split | Cell line characteristic | |
| --- | --- | --- | --- |
| | | PC1 | PC2 |
| 0 | T | -10,7497 | 2,527698 |
| 1 | T | -10.749746 | 2.5276984 |
| 2 | T | -10.749746 | 2.5276984 |
| 3 | T | 5.030760 | -8.6526055 |
| 4 | V | 5,03076 | -8,65261 |
| 5 | T | 5.030760 | -8.6526055 |
| 6 | T | 5.030760 | -8.6526055 |
| 7 | V | 4,663211 | 8,053145 |
| 8 | V | 4,663211 | 8,053145 |
| 9 | T | -10.749746 | 2.5276984 |
| 10 | T | -10.749746 | 2.5276984 |
| 11 | V | -5,84966 | 3,192198 |
| 12 | V | -5,84966 | 3,192198 |
| 13 | T | -6.508348 | -5.6802636 |
| 14 | T | -6.508348 | -5.6802636 |
| 15 | V | -6,50835 | -5,68026 |
| 16 | T | -6.046174 | 0.5393109 |
| 17 | T | 8.886373 | 2.3481203 |
| 18 | V | 8,886373 | 2,34812 |
| 19 | T | 8.886373 | 2.3481203 |
| 20 | T | 8.886373 | 2.3481203 |
| 21 | T | -6.046174 | 0.5393109 |
| 22 | V | -6,04617 | 0,539311 |
| 23 | T | 4.663211 | 8.0531453 |
| 24 | V | 4,663211 | 8,053145 |
| 25 | T | 4.663211 | 8.0531453 |
| 26 | T | 4.663211 | 8.0531453 |
| 27 | T | 4.663211 | 8.0531453 |

STEP 4. Standardization 4 for the training set

In this step, the matrices of cell lines and nanoforms characteristics (minimal particle size) should be standardized separately according to the following equation (Eq. 1). The standardized values of PC1, PC2 and nanoforms characteristics are reported in the Table A_4.

$$Z_{AX} = \frac{x_{AX} - m_X}{s_X} \qquad\qquad (Eq.1)$$

Where: $z_{AX}$ - standardized value of 'X feature' for the 'A object'; $x_{AX}$ - original value of 'X feature' for the 'A object'; mx - mean value of 'X feature' for all objects in the training set; sx - standard deviation of 'X feature' for all objects in the training set.

*Table A_4. Standardized values of PC1, PC2 and minimum particle size used in development and validation of method A- (T - training set, V - validation set)*

| ID | Split | Cell line characteristic | | Nanoform characteristic |
|---|---|---|---|---|
| | | PC1 | PC2 | Minimum particle size |
| 0 | T | -1,27504 | 0,307115 | -0,48453 |
| 1 | T | -1,27504 | 0,307115 | 0,119509 |
| 2 | T | -1,27504 | 0,307115 | 0,783631 |
| 3 | T | 0,782615 | -1,70827 | -0,56517 |
| 4 | V | 0,782615 | -1,70827 | -0,23469 |
| 5 | T | 0,782615 | -1,70827 | -0,2679 |
| 6 | T | 0,782615 | -1,70827 | 2,850318 |
| 7 | V | 0,73469 | 1,303144 | -0,46713 |
| 8 | V | 0,73469 | 1,303144 | 0,165365 |
| 9 | T | -1,27504 | 0,307115 | 0,086303 |
| 10 | T | -1,27504 | 0,307115 | 2,458169 |
| 11 | V | -0,63611 | 0,426899 | 0,114765 |
| 12 | V | -0,63611 | 0,426899 | -0,46871 |
| 13 | T | -0,722 | -1,17247 | -0,51457 |
| 14 | T | -0,722 | -1,17247 | -0,62526 |
| 15 | V | -0,722 | -1,17247 | -0,5462 |
| 16 | T | -0,66173 | -0,05132 | -0,5462 |
| 17 | T | 1,285358 | 0,274744 | 0,620763 |
| 18 | V | 1,285358 | 0,274744 | -0,05127 |
| 19 | T | 1,285358 | 0,274744 | -0,377 |
| 20 | T | 1,285358 | 0,274744 | -0,56991 |
| 21 | T | -0,66173 | -0,05132 | -0,59363 |
| 22 | V | -0,66173 | -0,05132 | -0,59363 |
| 23 | T | 0,73469 | 1,303144 | -0,5462 |
| 24 | V | 0,73469 | 1,303144 | 0,876925 |
| 25 | T | 0,73469 | 1,303144 | -0,60944 |
| 26 | T | 0,73469 | 1,303144 | -0,60944 |
| 27 | T | 0,73469 | 1,303144 | -0,60944 |

STEP 5. Model calibration 5 for the training set

Standardized values of PC1, PC2, nanoforms characteristics, and corresponding positive/negative mutagenic response of mammalian cells are used for QSAR model development using machine learning method: Support Vector Machines Classifier. The hyperparameters for reproducing the model should be as follows:

C=10, kernel='rbf', degree=3, gamma='scale', coef0=0.0, shrinking=True, probability=False, tol=0.001, cache_size=200, class_weight=None, verbose=False, max_iter=-1, decision_function_shape='ovr', break_ties=False, random_state=-None

At this step, a set of statistical parameters for the training set should be as below:

    Accuracy: 0.895
    Precision: 0.857
    Recall: 0.857
    F1_score: 0.857
    MCC: 0.774

STEP 6. Internal validation 6 for the training set

The internal validation of the developed model should be verified by performing stratified k-fold cross-validation. The hyperparameter should be as follow:
*cv: 5.*
At this step, a statistical parameter for training set cross-validation should be as follow:
Accuracy: 0.85

STEP 7. Validation set mapping 7

In this step, the validation set should be mapped (transformed) into principal components retaining all parameters from the L-PCA algorithm performed on the training set.

STEP 8. Validation set scaling 8

The validation set should be standardized by scaling the original values to the mean and standard deviation values calculated for the training set, according to the equation (Eq.2):

$$z_{BX} = \frac{x_{BX} - m_X}{s_X} \qquad\qquad (Eq.2)$$

Where: $z_{BX}$ - standardized value of 'X feature' for the 'B object' in the validation set; $x_{BX}$ - original value of 'X feature' for the 'B object' in the validation set; mx - mean value of 'X feature' for all objects in the training set; sx - standard deviation of 'X feature' for all objects in the training set.

STEP 9. Prediction 9 for the validation set

In this step the developed model is used to predict biological response for samples in the validation set.

STEP 10. External validation 10

For each sample in validation set, predicted values from step 9 should be compared with the experimental responses to calculate validation statistical parameters. The expected statistic values for the validation set should be as follow:

    Accuracy: 0.778
    Precision: 0.667
    Recall: 0.667
    F1_score: 0.667
    MCC: 0.5

STEP 11. New samples defining 11

In this step, new samples are defined through specification of i) minimal particle size as a numerical value within the range indicated below, and ii) cell line characeristics through adherence to the specific group, in a binary scale, i.e.: 1 - yes, 0 - no. To ensure high reliability of predictions, the provided values of input parameters should be within the applicability domain defined below. The order of input parameters should be the same as in the training set.

Cell line chracteristics:

- Cell line origin:

  ○ human [1 or 0]

- Cell type and organ of origin:

  ○ epithelium lung [1 or 0]
  ○ fibroblast adipose tissue [1 or 0]
  ○ mononuclear cell peripheral blood (lymphocytes, monocytes)
  ○ epithelium colon
  ○ endothelium umbilical vein

- Germ layer the cells originate from:

  ○ endoderm [1 or 0]

- Biological nature of cell used:

  ○ cancer [1 or 0]

Nanoform characteristic:

- Minimal size - minimum size of nanoparticle in the sample, quantitative factor, derived from TEM images: 5 - 224.8 [nm]

STEP 12. New samples mapping 12

In this step, new samples should be mapped (transformed) into principal components retaining all parameters from the L-PCA algorithm performed on the training set.

STEP 13. New samples scaling 13

The input parameters should be standardized by scaling the original values to the mean and standard deviation values calculated for the training set, according to the equation (Eq.3):

$$z_{CX} = \frac{x_{CX} - m_X}{s_X} \qquad\qquad (Eq.3)$$

Where: $z_{CX}$ - standardized value of 'X feature' for the input 'C sample'; $x_{BX}$ - original value of 'X feature' for the input 'C sample'; mx - mean value of 'X feature' for all objects in the training set; sx - standard deviation of 'X feature' for all objects in the training set.

STEP 14. Predicting 14 mutagenic effects for the new sample

In this step the developed model is used to predict biological response for the new samples - new, untested nanoform(s) and defined cell line(s). As a result, a positive or negative mutagenic response is assigned to the defined samples.

**Prediction case study of method A**

The presented case study is carried out on new, untested $SiO_2$ nanoforms, and defined cell line, for which the induction of the mutagenic effect is unknown. Predicting the unknown activity requires performing all 14 steps included in method A and shown in Figure 1. The algorithm can be executed by means of any programming language and/or data analysis software.

After performing steps 1-10 of method A completed with the correctly reconstructed QSAR model, confirmed by the convergence of statistical parameters for training and validation set with these presented in this document; a new $SiO_2$

nanoform and cell line (A549) was defined in step 11:

Cell line characteristics:

- cell line origin:

    ○ human: 1

- cell type and organ of origin:

    ○ epithelium lung: 1
    ○ fibroblast adipose tissue: 0
    ○ mononuclear cell peripheral blood (lymphocytes, monocytes): 0
    ○ epithelium colon: 0
    ○ endothelium umbilical vein: 0

- germ layers the cells originate from:

    ○ endoderm: 1

- biological nature of cell used:

    ○ normal: 0

    ○ cancer: 1

Nanoform characteristics:

- Minimal size - minimum size of nanoparticle in the sample, quantitative factor, derived from TEM images: 15 nm

Subsequently, the newly defined sample from the example underwent mapping in step 12 (only cell line characteristics) and scaling in step 13 (both cell line characteristic and minimal particle size of $SiO_2$). The obtained results are as follows (Table 1):

Table 1. Inputs of the newly defined sample in Example_1 for the predictions with the QSAR model in method A.

| Sample ID | Cell line characteristic | | Nanoform characteristic |
|---|---|---|---|
| | PC1 | PC2 | Minimum particle size |
| Example_1 | -1.275044 | 0.307115 | -0.467133 |

In the last 14 step, the scaled score values of PC1, PC2 for cell line characteristics, and scaled minimum particle size of $SiO_2$, were used as inputs to make predictions with the QSAR model in method A: support vector machine classifier. The application of a recreated A model resulted in: <u>negative response</u> of mutagenic effect for the newly defined sample of example.

**METHOD B - QSAR model for genotoxicity of $SiO_2$ nanoparticles expressed with *in vitro* comet assay.**

Method B employs a battery of common data curation and machine learning algorithms: dataset splitting based on random split algorithm, dimensionality reduction based on logistic principal component analysis, standardization, and model development based on Decision Tree Classifier. Said algorithms can be executed by means of any programming language and/or data analysis software.

The general scheme to recreate method B of the invention is presented in Figure 1. The procedure is divided into 14 steps described below:

STEP 1. Datasets preparation 1.

Method B is based on an *in vitro* experimental data available for limited number of samples described with: i) cell lines and nanoforms characterization (Table B_1), and ii) observed genotoxic response (Table B_2). The observed responses for the available samples are as follows:

[positive response] 43 samples
[negative response] 22 samples

Table B_1. Cell line characteristics and SiO$_2$ nanoform characteristic of samples used in development and validation of method B (T – training set, V – validation set)

| ID | Cell line | Split | | | | | | | | | | | | | | | | | | | | | | Nanoform characteristic |
|---|---|---|---|---|---|---|---|---|---|---|---|---|---|---|---|---|---|---|---|---|---|---|---|---|
| | | | Human | Mouse | Hamster | Rat | Fibroblast embryo | Epithelium Colon | Epithelium lung | Neuroblasts brain | Keranotites skin | Mononuclear cell peripheral blood | Endothelium umbilical vein brain | Lymphoblast spleen | Epithalium ovary | Fibroblast lung | Macrophage monocytes | Epithelial kidney | Embryo kidney | Cancer | Ectoderm | Mesoderm | Endoderm | Minimum particle size |
| 0 | 3T3-L1 | T | 0 | 1 | 0 | 0 | 1 | 0 | 0 | 0 | 0 | 0 | 0 | 0 | 0 | 0 | 0 | 0 | 0 | 0 | 0 | 1 | 0 | 33.21 |
| 1 | 3T3-L1 | T | 0 | 1 | 0 | 0 | 1 | 0 | 0 | 0 | 0 | 0 | 0 | 0 | 0 | 0 | 0 | 0 | 0 | 0 | 0 | 1 | 0 | 34.89 |
| 2 | 3T3-L1 | T | 0 | 1 | 0 | 0 | 1 | 0 | 0 | 0 | 0 | 0 | 0 | 0 | 0 | 0 | 0 | 0 | 0 | 0 | 0 | 1 | 0 | 21.32 |
| 3 | 3T3-L1 | T | 0 | 1 | 0 | 0 | 1 | 0 | 0 | 0 | 0 | 0 | 0 | 0 | 0 | 0 | 0 | 0 | 0 | 0 | 0 | 1 | 0 | 30.51 |
| 4 | Caco-2 | T | 1 | 0 | 0 | 0 | 0 | 1 | 0 | 0 | 0 | 0 | 0 | 0 | 0 | 0 | 0 | 0 | 0 | 1 | 0 | 0 | 1 | 14 |
| 5 | Primary mouse embryo fibroblasts | T | 0 | 1 | 0 | 0 | 1 | 0 | 0 | 0 | 0 | 0 | 0 | 0 | 0 | 0 | 0 | 0 | 0 | 0 | 0 | 1 | 0 | 13.6 |
| 6 | A549 | T | 1 | 0 | 0 | 0 | 0 | 0 | 1 | 0 | 0 | 0 | 0 | 0 | 0 | 0 | 0 | 0 | 0 | 1 | 0 | 0 | 1 | 16 |
| 7 | A549 | V | 1 | 0 | 0 | 0 | 0 | 0 | 1 | 0 | 0 | 0 | 0 | 0 | 0 | 0 | 0 | 0 | 0 | 1 | 0 | 0 | 1 | 60 |
| 8 | SH-SY5Y | V | 1 | 0 | 0 | 0 | 0 | 0 | 0 | 1 | 0 | 0 | 0 | 0 | 0 | 0 | 0 | 0 | 0 | 1 | 1 | 0 | 0 | 14.1 |
| 9 | SH-SY5Y | T | 1 | 0 | 0 | 0 | 0 | 0 | 0 | 1 | 0 | 0 | 0 | 0 | 0 | 0 | 0 | 0 | 0 | 1 | 1 | 0 | 0 | 13.3 |
| 10 | HaCaT | T | 1 | 0 | 0 | 0 | 0 | 0 | 0 | 0 | 1 | 0 | 0 | 0 | 0 | 0 | 0 | 0 | 0 | 0 | 1 | 0 | 0 | 14.3 |
| 11 | HaCaT | T | 1 | 0 | 0 | 0 | 0 | 0 | 0 | 0 | 1 | 0 | 0 | 0 | 0 | 0 | 0 | 0 | 0 | 0 | 1 | 0 | 0 | 18.7 |
| 12 | HaCaT | T | 1 | 0 | 0 | 0 | 0 | 0 | 0 | 0 | 1 | 0 | 0 | 0 | 0 | 0 | 0 | 0 | 0 | 0 | 1 | 0 | 0 | 166.1 |
| 13 | A549 | T | 1 | 0 | 0 | 0 | 0 | 0 | 1 | 0 | 0 | 0 | 0 | 0 | 0 | 0 | 0 | 0 | 0 | 1 | 0 | 0 | 1 | 5 |
| 14 | BEAS-2B | T | 1 | 0 | 0 | 0 | 0 | 0 | 1 | 0 | 0 | 0 | 0 | 0 | 0 | 0 | 0 | 0 | 0 | 0 | 0 | 0 | 1 | 5 |
| 15 | A549 | T | 1 | 0 | 0 | 0 | 0 | 0 | 1 | 0 | 0 | 0 | 0 | 0 | 0 | 0 | 0 | 0 | 0 | 1 | 0 | 0 | 1 | 30 |
| 16 | HT29 | T | 1 | 0 | 0 | 0 | 0 | 1 | 0 | 0 | 0 | 0 | 0 | 0 | 0 | 0 | 0 | 0 | 0 | 1 | 0 | 0 | 1 | 30 |
| 17 | HaCaT | T | 1 | 0 | 0 | 0 | 0 | 0 | 0 | 0 | 1 | 0 | 0 | 0 | 0 | 0 | 0 | 0 | 0 | 0 | 1 | 0 | 0 | 30 |
| 18 | HaCaT | T | 1 | 0 | 0 | 0 | 0 | 0 | 0 | 0 | 1 | 0 | 0 | 0 | 0 | 0 | 0 | 0 | 0 | 0 | 1 | 0 | 0 | 64.2 |
| 19 | HaCaT | T | 1 | 0 | 0 | 0 | 0 | 0 | 0 | 0 | 1 | 0 | 0 | 0 | 0 | 0 | 0 | 0 | 0 | 0 | 1 | 0 | 0 | 64.2 |
| 20 | HaCaT | V | 1 | 0 | 0 | 0 | 0 | 0 | 0 | 0 | 1 | 0 | 0 | 0 | 0 | 0 | 0 | 0 | 0 | 0 | 1 | 0 | 0 | 64.2 |
| 21 | HEK293 | T | 1 | 0 | 0 | 0 | 0 | 0 | 0 | 0 | 0 | 0 | 0 | 0 | 0 | 0 | 0 | 1 | 1 | 0 | 0 | 1 | 0 | 5.4 |
| 22 | HEK293 | T | 1 | 0 | 0 | 0 | 0 | 0 | 0 | 0 | 0 | 0 | 0 | 0 | 0 | 0 | 0 | 1 | 1 | 0 | 0 | 1 | 0 | 14.1 |
| 23 | HEK293 | T | 1 | 0 | 0 | 0 | 0 | 0 | 0 | 0 | 0 | 0 | 0 | 0 | 0 | 0 | 0 | 1 | 1 | 0 | 0 | 1 | 0 | 29.6 |
| 24 | HEK293 | V | 1 | 0 | 0 | 0 | 0 | 0 | 0 | 0 | 0 | 0 | 0 | 0 | 0 | 0 | 0 | 1 | 1 | 0 | 0 | 1 | 0 | 54.03 |
| 25 | Human lymphocytes | T | 1 | 0 | 0 | 0 | 0 | 0 | 0 | 0 | 0 | 1 | 0 | 0 | 0 | 0 | 0 | 0 | 0 | 0 | 0 | 1 | 0 | 5.4 |
| 26 | Human lymphocytes | T | 1 | 0 | 0 | 0 | 0 | 0 | 0 | 0 | 0 | 1 | 0 | 0 | 0 | 0 | 0 | 0 | 0 | 0 | 0 | 1 | 0 | 14.1 |

EP 4 485 467 A1

| | | | | | | | | | | | | | | | | | | | | | | | |
|---|---|---|---|---|---|---|---|---|---|---|---|---|---|---|---|---|---|---|---|---|---|---|---|
| 27 | Human lymphocytes | T | 1 | 0 | 0 | 0 | 0 | 0 | 0 | 0 | 0 | 1 | 0 | 0 | 0 | 0 | 0 | 0 | 0 | 0 | 0 | 1 | 0 | 29.6 |
| 28 | Human lymphocytes | T | 1 | 0 | 0 | 0 | 0 | 0 | 0 | 0 | 0 | 1 | 0 | 0 | 0 | 0 | 0 | 0 | 0 | 0 | 0 | 1 | 0 | 54.03 |
| 29 | HUVEC | V | 1 | 0 | 0 | 0 | 0 | 0 | 0 | 0 | 0 | 0 | 1 | 0 | 0 | 0 | 0 | 0 | 0 | 0 | 0 | 1 | 0 | 62 |
| 30 | HT29 | V | 1 | 0 | 0 | 0 | 0 | 1 | 0 | 0 | 0 | 0 | 0 | 0 | 0 | 0 | 0 | 0 | 0 | 1 | 0 | 0 | 1 | 12 |
| 31 | HT29 | T | 1 | 0 | 0 | 0 | 0 | 1 | 0 | 0 | 0 | 0 | 0 | 0 | 0 | 0 | 0 | 0 | 0 | 1 | 0 | 0 | 1 | 40 |
| 32 | Human lymphocytes | V | 1 | 0 | 0 | 0 | 0 | 0 | 0 | 0 | 0 | 1 | 0 | 0 | 0 | 0 | 0 | 0 | 0 | 0 | 0 | 1 | 0 | 10 |
| 33 | Human lymphocytes | T | 1 | 0 | 0 | 0 | 0 | 0 | 0 | 0 | 0 | 1 | 0 | 0 | 0 | 0 | 0 | 0 | 0 | 0 | 0 | 1 | 0 | 10 |
| 34 | Human lymphocytes | T | 1 | 0 | 0 | 0 | 0 | 0 | 0 | 0 | 0 | 1 | 0 | 0 | 0 | 0 | 0 | 0 | 0 | 0 | 0 | 1 | 0 | 10 |
| 35 | Human lymphocytes | T | 1 | 0 | 0 | 0 | 0 | 0 | 0 | 0 | 0 | 1 | 0 | 0 | 0 | 0 | 0 | 0 | 0 | 0 | 0 | 1 | 0 | 6 |
| 36 | Human lymphocytes | T | 1 | 0 | 0 | 0 | 0 | 0 | 0 | 0 | 0 | 1 | 0 | 0 | 0 | 0 | 0 | 0 | 0 | 0 | 0 | 1 | 0 | 20 |
| 37 | Human lymphocytes | V | 1 | 0 | 0 | 0 | 0 | 0 | 0 | 0 | 0 | 1 | 0 | 0 | 0 | 0 | 0 | 0 | 0 | 0 | 0 | 1 | 0 | 50 |
| 38 | TK6 | T | 1 | 0 | 0 | 0 | 0 | 0 | 0 | 0 | 0 | 0 | 0 | 1 | 0 | 0 | 0 | 0 | 1 | 0 | 1 | 0 | 14.7 |
| 39 | II9T3 | V | 0 | 0 | 1 | 0 | 0 | 0 | 0 | 0 | 0 | 0 | 0 | 0 | 1 | 0 | 0 | 0 | 0 | 0 | 0 | 1 | 0 | 14.7 |
| 40 | A549 | T | 1 | 0 | 0 | 0 | 0 | 0 | 1 | 0 | 0 | 0 | 0 | 0 | 0 | 0 | 0 | 0 | 0 | 1 | 0 | 0 | 1 | 50 |
| 41 | A549 | T | 1 | 0 | 0 | 0 | 0 | 0 | 1 | 0 | 0 | 0 | 0 | 0 | 0 | 0 | 0 | 0 | 0 | 1 | 0 | 0 | 1 | 10 |
| 42 | V79 | V | 0 | 0 | 1 | 0 | 0 | 0 | 0 | 0 | 0 | 0 | 0 | 0 | 0 | 1 | 0 | 0 | 0 | 0 | 0 | 1 | 0 | 15 |
| 43 | V79 | V | 0 | 0 | 1 | 0 | 0 | 0 | 0 | 0 | 0 | 0 | 0 | 0 | 0 | 1 | 0 | 0 | 0 | 0 | 0 | 1 | 0 | 55 |
| 44 | Human lymphocytes | T | 1 | 0 | 0 | 0 | 0 | 0 | 0 | 0 | 0 | 1 | 0 | 0 | 0 | 0 | 0 | 0 | 0 | 0 | 0 | 1 | 0 | 15 |
| 45 | RAW 264.70 | T | 0 | 1 | 0 | 0 | 0 | 0 | 0 | 0 | 0 | 0 | 0 | 0 | 0 | 0 | 1 | 0 | 0 | 1 | 0 | 1 | 0 | 8 |
| 46 | A549 | T | 1 | 0 | 0 | 0 | 0 | 0 | 1 | 0 | 0 | 0 | 0 | 0 | 0 | 0 | 0 | 0 | 0 | 1 | 0 | 0 | 1 | 62 |
| 47 | HUVEC | V | 1 | 0 | 0 | 0 | 0 | 0 | 0 | 0 | 0 | 0 | 1 | 0 | 0 | 0 | 0 | 0 | 0 | 0 | 0 | 1 | 0 | 52.72 |
| 48 | HaCaT | T | 1 | 0 | 0 | 0 | 0 | 0 | 0 | 0 | 1 | 0 | 0 | 0 | 0 | 0 | 0 | 0 | 0 | 0 | 1 | 0 | 0 | 15 |
| 49 | Human bronchial models | T | 1 | 0 | 0 | 0 | 0 | 0 | 0 | 0 | 0 | 0 | 0 | 0 | 0 | 0 | 0 | 0 | 0 | 0 | 0 | 0 | 1 | 15 |
| 50 | A549 | T | 1 | 0 | 0 | 0 | 0 | 0 | 1 | 0 | 0 | 0 | 0 | 0 | 0 | 0 | 0 | 0 | 0 | 1 | 0 | 0 | 1 | 12.32 |
| 51 | BEAS-2B | T | 1 | 0 | 0 | 0 | 0 | 0 | 1 | 0 | 0 | 0 | 0 | 0 | 0 | 0 | 0 | 0 | 0 | 0 | 0 | 0 | 1 | 12.32 |
| 52 | A549 | V | 1 | 0 | 0 | 0 | 0 | 0 | 1 | 0 | 0 | 0 | 0 | 0 | 0 | 0 | 0 | 0 | 0 | 1 | 0 | 0 | 1 | 14.88 |
| 53 | BEAS-2B | T | 1 | 0 | 0 | 0 | 0 | 0 | 1 | 0 | 0 | 0 | 0 | 0 | 0 | 0 | 0 | 0 | 0 | 0 | 0 | 0 | 1 | 14.88 |
| 54 | TH1 cells | T | 1 | 0 | 0 | 0 | 0 | 0 | 0 | 0 | 0 | 0 | 0 | 0 | 0 | 0 | 1 | 0 | 0 | 0 | 0 | 1 | 0 | 23.8 |
| 55 | HUVEC | T | 1 | 0 | 0 | 0 | 0 | 0 | 0 | 0 | 0 | 0 | 1 | 0 | 0 | 0 | 0 | 0 | 0 | 0 | 0 | 1 | 0 | 83.8 |
| 56 | HUVEC | T | 1 | 0 | 0 | 0 | 0 | 0 | 0 | 0 | 0 | 0 | 1 | 0 | 0 | 0 | 0 | 0 | 0 | 0 | 0 | 1 | 0 | 11.3 |
| 57 | HUVEC | T | 1 | 0 | 0 | 0 | 0 | 0 | 0 | 0 | 0 | 0 | 1 | 0 | 0 | 0 | 0 | 0 | 0 | 0 | 0 | 1 | 0 | 20.7 |
| 58 | HUVEC | T | 1 | 0 | 0 | 0 | 0 | 0 | 0 | 0 | 0 | 0 | 1 | 0 | 0 | 0 | 0 | 0 | 0 | 0 | 0 | 1 | 0 | 8.5 |
| 59 | RAW 264.70 | T | 0 | 1 | 0 | 0 | 0 | 0 | 0 | 0 | 0 | 0 | 0 | 0 | 0 | 0 | 1 | 0 | 0 | 1 | 0 | 1 | 0 | 15 |
| 60 | RAW 264.70 | T | 0 | 1 | 0 | 0 | 0 | 0 | 0 | 0 | 0 | 0 | 0 | 0 | 0 | 0 | 1 | 0 | 0 | 1 | 0 | 1 | 0 | 12.1 |
| 61 | RAW 264.70 | T | 0 | 0 | 1 | 0 | 0 | 0 | 0 | 0 | 0 | 0 | 0 | 0 | 0 | 1 | 0 | 0 | 0 | 0 | 0 | 1 | 0 | 13.49 |
| 62 | A549 | T | 1 | 0 | 0 | 0 | 0 | 0 | 1 | 0 | 0 | 0 | 0 | 0 | 0 | 0 | 0 | 0 | 0 | 1 | 0 | 0 | 1 | 58 |
| 63 | BEAS-2B | T | 1 | 0 | 0 | 0 | 0 | 0 | 1 | 0 | 0 | 0 | 0 | 0 | 0 | 0 | 0 | 0 | 0 | 0 | 0 | 0 | 1 | 7 |
| 64 | BEAS-2B | T | 1 | 0 | 0 | 0 | 0 | 0 | 1 | 0 | 0 | 0 | 0 | 0 | 0 | 0 | 0 | 0 | 0 | 0 | 0 | 0 | 1 | 7 |

*Table B_2. Mutagenic response of samples used in development and validation of method B (1 - positive, 0 - negative response) (T - training set, V - validation s*

| ID | Split | Mutagenic effect |
|---|---|---|
| 0 | T | 0 |
| 1 | T | 0 |
| 2 | T | 0 |
| 3 | T | 0 |
| 4 | T | 0 |
| 5 | T | 1 |
| 6 | T | 0 |
| 7 | V | 0 |
| 8 | V | 1 |
| 9 | T | 1 |
| 10 | T | 1 |
| 11 | T | 1 |
| 12 | T | 1 |
| 13 | T | 0 |
| 14 | T | 0 |
| 15 | T | 1 |
| 16 | T | 1 |
| 17 | T | 1 |
| 18 | T | 1 |
| 19 | T | 1 |
| 20 | V | 1 |
| 21 | T | 1 |
| 22 | T | 1 |
| 23 | T | 1 |
| 24 | V | 1 |
| 25 | T | 1 |
| 26 | T | 1 |
| 27 | T | 1 |
| 28 | T | 1 |
| 29 | V | 1 |
| 30 | V | 1 |
| 31 | T | 0 |
| 32 | V | 0 |
| 33 | T | 0 |
| 34 | T | 0 |
| 35 | T | 1 |
| 36 | T | 1 |
| 37 | V | 1 |
| 38 | T | 0 |

(continued)

| ID | Split | Mutagenic effect |
|---|---|---|
| 39 | V | 0 |
| 40 | T | 1 |
| 41 | T | 0 |
| 42 | V | 1 |
| 43 | V | 1 |
| 44 | T | 0 |
| 45 | T | 0 |
| 46 | T | 0 |
| 47 | V | 0 |
| 48 | T | 1 |
| 49 | T | 1 |
| 50 | T | 1 |
| 51 | T | 1 |
| 52 | V | 1 |
| 53 | T | 1 |
| 54 | T | 0 |
| 55 | T | 1 |
| 56 | T | 1 |
| 57 | T | 1 |
| 58 | T | 1 |
| 59 | T | 1 |
| 60 | T | 1 |
| 61 | T | 1 |
| 62 | T | 1 |
| 63 | T | 0 |
| 64 | T | 1 |

STEP 2. Datasets splitting 2 into a training set and a validation set.

The recreation of the B model requires the same split into training and validation sets as it is available in Table B_1. The decisive split was achieved through random split algorithm (test_size=0.2, random_state=42, stratify=y). The training set is used for calibration and verification of the model robustness (internal validation). The validation set is used for verification of the model prediction ability (external validation). The ratio between number of samples in the training and validation set is 8:2 (52 training set: 13 validation set).
The number of samples with positive/negative responses in training set is as follows:

[positive response] 34 samples
[negative response] 18 samples

The number of samples with positive/negative responses in validation set is as follows:

[positive response] 9 samples
[negative response] 4 samples

STEP 3. L-PCA 3 for the training set

In logistic principal component analysis (L-PCA), the multidimensionality of the matrix of cell line characteristic is reduced to two first principal components (PCs: PC1, PC2), which explain 73% of the overall deviance.

The hyperparameters for L-PCA are as follows:

k=2, m=9, quiet=TRUE, partial_decomp=FALSE, max_iters=1000, conv_criteria=1e-5, random_start=FALSE, main_effects=TRUE.

Obtained scores values for samples in training set are reported in the Table B_3.

*Table B_3. Scores values of samples used in development and validation of method β- (T - training set, V - validation set).*

| ID | Split | Cell line characteristic | |
|---|---|---|---|
| | | PC1 | PC2 |
| 0 | T | -18,0651 | 5,735649 |
| 1 | T | -18,0651 | 5,735649 |
| 2 | T | -18,0651 | 5,735649 |
| 3 | T | -18,0651 | 5,735649 |
| 4 | T | 16,40126 | 11,90374 |
| 5 | T | -18,0651 | 5,735649 |
| 6 | T | 21,92406 | 7,284336 |
| 7 | V | 21,92406 | 7,284336 |
| 8 | V | 14,28199 | -3,00942 |
| 9 | T | 14,28199 | -3,00942 |
| 10 | T | 13,67441 | -16,58 |
| 11 | T | 13,67441 | -16,58 |
| 12 | T | 13,67441 | -16,58 |
| 13 | T | 21,92406 | 7,284336 |
| 14 | T | 19,82173 | -0,42913 |
| 15 | T | 21,92406 | 7,284336 |
| 16 | T | 16,40126 | 11,90374 |
| 17 | T | 13,67441 | -16,58 |
| 18 | T | 13,67441 | -16,58 |
| 19 | T | 13,67441 | -16,58 |
| 20 | V | 13,67441 | -16,58 |
| 21 | T | -5,56865 | -12,214 |
| 22 | T | -5,56865 | -12,214 |
| 23 | T | -5,56865 | -12,214 |
| 24 | V | -5,56865 | -12,214 |
| 25 | T | -6,08407 | -10,9538 |
| 26 | T | -6,08407 | -10,9538 |
| 27 | T | -6,08407 | -10,9538 |
| 28 | T | -6,08407 | -10,9538 |
| 29 | V | -1,55019 | -4,79095 |
| 30 | V | 16,40126 | 11,90374 |
| 31 | T | 16,40126 | 11,90374 |

(continued)

| ID | Split | Cell line characteristic | |
|----|-------|------|------|
| | | PC1 | PC2 |
| 32 | V | -6,08407 | -10,9538 |
| 33 | T | -6,08407 | -10,9538 |
| 34 | T | -6,08407 | -10,9538 |
| 35 | T | -6,08407 | -10,9538 |
| 36 | T | -6,08407 | -10,9538 |
| 37 | V | -6,08407 | -10,9538 |
| 38 | T | 1,257512 | 5,26296 |
| 39 | V | -7,35454 | 1,562088 |
| 40 | T | 21,92406 | 7,284336 |
| 41 | T | 21,92406 | 7,284336 |
| 42 | V | -8,67476 | 2,622365 |
| 43 | V | -8,67476 | 2,622365 |
| 44 | T | -6,08407 | -10,9538 |
| 45 | T | -9,32348 | 18,00062 |
| 46 | T | 21,92406 | 7,284336 |
| 47 | V | -1,55019 | -4,79095 |
| 48 | T | 13,67441 | -16,58 |
| 49 | T | 12,67158 | -0,80015 |
| 50 | T | 21,92406 | 7,284336 |
| 51 | T | 19,82173 | -0,42913 |
| 52 | V | 21,92406 | 7,284336 |
| 53 | T | 19,82173 | -0,42913 |
| 54 | T | -3,28962 | -8,20027 |
| 55 | T | -1,55019 | -4,79095 |
| 56 | T | -1,55019 | -4,79095 |
| 57 | T | -1,55019 | -4,79095 |
| 58 | T | -1,55019 | -4,79095 |
| 59 | T | -9,32348 | 18,00062 |
| 60 | T | -9,32348 | 18,00062 |
| 61 | T | -8,67476 | 2,622365 |
| 62 | T | 21,92406 | 7,284336 |
| 63 | T | 19,82173 | -0,42913 |
| 64 | T | 19,82173 | -0,42913 |

STEP 4. Standardization 4 for the training set

In this step, the matrices of cell lines and nanoforms characteristics (minimal particle size) should be standardized separately according to the following equation (Eq.1). The standardized values of PC1, PC2 and nanoforms characteristics are reported in the Table B_4.

$$z_{AX} = \frac{x_{AX} - m_X}{s_X} \qquad\qquad\qquad (Eq.1)$$

Where: $z_{AX}$ - standardized value of 'X feature' for the 'A object'; $x_{AX}$ - original value of 'X feature' for the 'A object'; mx - mean value of 'X feature' for all objects in the training set; sx - standard deviation of 'X feature' for all objects in the training set.

*Table B_4. Standardized values of PC1, PC2 and minimum particle size used in development and validation of method β- (T - training set, V - validation set)*

| ID | Split | Cell line characteristic | | Nanoform characteristic |
|---|---|---|---|---|
| | | PC1 | PC2 | Minimum particle size |
| 0 | T | -1,66873 | 0,748232 | 0,284248 |
| 1 | T | -1,66873 | 0,748232 | 0,347202 |
| 2 | T | -1,66873 | 0,748232 | -0,1613 |
| 3 | T | -1,66873 | 0,748232 | 0,183073 |
| 4 | T | 0,867768 | 1,350752 | -0,43559 |
| 5 | T | -1,66873 | 0,748232 | -0,45058 |
| 6 | T | 1,27421 | 0,899513 | -0,36065 |
| 7 | V | 1,27421 | 0,899513 | 1,288129 |
| 8 | V | 0,711804 | -0,10602 | -0,43185 |
| 9 | T | 0,711804 | -0,10602 | -0,46182 |
| 10 | T | 0,66709 | -1,43163 | -0,42435 |
| 11 | T | 0,66709 | -1,43163 | -0,25947 |
| 12 | T | 0,66709 | -1,43163 | 5,263931 |
| 13 | T | 1,27421 | 0,899513 | -0,77284 |
| 14 | T | 1,119492 | 0,146035 | -0,77284 |
| 15 | T | 1,27421 | 0,899513 | 0,163963 |
| 16 | T | 0,867768 | 1,350752 | 0,163963 |
| 17 | T | 0,66709 | -1,43163 | 0,163963 |
| 18 | T | 0,66709 | -1,43163 | 1,445512 |
| 19 | T | 0,66709 | -1,43163 | 1,445512 |
| 20 | V | 0,66709 | -1,43163 | 1,445512 |
| 21 | T | -0,74907 | -1,00515 | -0,75785 |
| 22 | T | -0,74907 | -1,00515 | -0,43185 |
| 23 | T | -0,74907 | -1,00515 | 0,148974 |
| 24 | V | -0,74907 | -1,00515 | 1,06442 |
| 25 | T | -0,787 | -0,88205 | -0,75785 |
| 26 | T | -0,787 | -0,88205 | -0,43185 |
| 27 | T | -0,787 | -0,88205 | 0,148974 |
| 28 | T | -0,787 | -0,88205 | 1,06442 |
| 29 | V | -0,45334 | -0,28004 | 1,363073 |
| 30 | V | 0,867768 | 1,350752 | -0,51054 |
| 31 | T | 0,867768 | 1,350752 | 0,538685 |
| 32 | V | -0,787 | -0,88205 | -0,58548 |

(continued)

| ID | Split | Cell line characteristic | | Nanoform characteristic |
| --- | --- | --- | --- | --- |
| | | PC1 | PC2 | Minimum particle size |
| 33 | T | -0,787 | -0,88205 | -0,58548 |
| 34 | T | -0,787 | -0,88205 | -0,58548 |
| 35 | T | -0,787 | -0,88205 | -0,73537 |
| 36 | T | -0,787 | -0,88205 | -0,21076 |
| 37 | V | -0,787 | -0,88205 | 0,913407 |
| 38 | T | -0,24671 | 0,702058 | -0,40936 |
| 39 | V | -0,8805 | 0,340544 | -0,40936 |
| 40 | T | 1,27421 | 0,899513 | 0,913407 |
| 41 | T | 1,27421 | 0,899513 | -0,58548 |
| 42 | V | -0,97766 | 0,444115 | -0,39812 |
| 43 | V | -0,97766 | 0,444115 | 1,100768 |
| 44 | T | -0,787 | -0,88205 | -0,39812 |
| 45 | T | -1,0254 | 1,946316 | -0,66043 |
| 46 | T | 1,27421 | 0,899513 | 1,363073 |
| 47 | V | -0,45334 | -0,28004 | 1,015331 |
| 48 | T | 0,66709 | -1,43163 | -0,39812 |
| 49 | T | 0,593289 | 0,109792 | -0,39812 |
| 50 | T | 1,27421 | 0,899513 | -0,49855 |
| 51 | T | 1,119492 | 0,146035 | -0,49855 |
| 52 | V | 1,27421 | 0,899513 | -0,40262 |
| 53 | T | 1,119492 | 0,146035 | -0,40262 |
| 54 | T | -0,58135 | -0,61308 | -0,06837 |
| 55 | T | -0,45334 | -0,28004 | 2,179968 |
| 56 | T | -0,45334 | -0,28004 | -0,53677 |
| 57 | T | -0,45334 | -0,28004 | -0,18453 |
| 58 | T | -0,45334 | -0,28004 | -0,64169 |
| 59 | T | -1,0254 | 1,946316 | -0,39812 |
| 60 | T | -1,0254 | 1,946316 | -0,50679 |
| 61 | T | -0,97766 | 0,444115 | -0,4547 |
| 62 | T | 1,27421 | 0,899513 | 1,213185 |
| 63 | T | 1,119492 | 0,146035 | -0,6979 |
| 64 | T | 1,119492 | 0,146035 | -0,6979 |

STEP 5. Model calibration 5 for the training set

Standardized values of PC1, PC2, nanoforms characteristics, and corresponding positive/negative mutagenic response of mammalian cells are used for QSAR model development using machine learning method: Decision Tree Classifier. The hyperparameters for reproducing the model should be as follows:
(criterion='gini', splitter='best', max_depth=4, min_samples_split=2, min_samples_leaf=1, mi n_weight_fraction_leaf=0.0, max_features=None, random_state=None, max_leaf_nodes=Non e, min_impurity_decrease=0.0,class_weight=balanced, ccp_alpha=0.0)

At this step, a set of statistical parameters for training set should be as below:

Accuracy: 0.865
Precision: 0.966
Recall: 0.824
F1_score: 0.889
MCC: 0.736

STEP 6. Internal validation 6 for the training set

The internal validation of the developed model should be verified by performing stratified k-fold cross-validation. The hyperparameter should be as follow:
*cv: 5*
At this step, a set of statistical parameters for training set cross-validation should be as follow: Accuracy: 0.678

STEP 7. Validation set mapping 7.

In this step, the validation set should be mapped (transformed) into principal components retaining all parameters from the L-PCA algorithm performed on the training set.

STEP 8. Validation set scaling 8.

The validation set should be standardized by scaling the original values to the mean and standard deviation values calculated for the training set, according to the equation (Eq.2):

$$z_{BX} = \frac{x_{BX} - m_X}{s_X} \qquad\qquad (Eq.2)$$

Where: $z_{BX}$ - standardized value of 'X feature' for the 'B object' in the validation set; $x_{BX}$ original value of 'X feature' for the 'B object' in the validation set; mx - mean value of 'X feature' for all objects in the training set; sx - standard deviation of 'X feature' for all objects in the training set.

STEP 9. Prediction 9 for the validation set

In this step the developed model is used to predict biological response for samples in the validation set.

STEP 10. External validation 10.

For each sample in validation set, predicted values from step 9 should be compared with the experimental responses to calculate validation statistical parameters. The expected statistic values for the validation set should be as follow:

Accuracy: 0.769

Precision: 0.8

Recall: 0.889

F1_score: 0.842

MCC: 0.426

STEP 11. New samples defining 11.

In this step, new samples are defined through specification of i) minimal particle size as a numerical value within the range indicated below, and ii) cell line characeristics through adherence to the specific group, in a binary scale, i.e.: 1 - yes, 0 - no. To ensure high reliability of predictions, the provided values of input parameters should be within the applicability domain defined below. The order of input parameters should be the same as in the training set.

Cell line chracteristics:

- Cell line origin:

  ○ human [1 or 0]
  ○ mouse [1 or 0]
  ○ hamster [1 or 0]
  ○ rat [1 or 0]

- Cell type and organ of origin:

  ○ fibroblast embryo [1 or 0]
  ○ epithelium colon [1 or 0]
  ○ epithelium lung [1 or 0]
  ○ neuroblasts brain [1 or 0]
  ○ keratinocytes skin [1 or 0]
  ○ mononuclear cell peripheral blood (lymphocytes, monocytes) [1 or 0] ○ endothelium umbilical vein [1 or 0]
  ○ lymphoblast spleen [1 or 0]
  ○ epithelium ovary [1 or 0]
  ○ fibroblast lung [1 or 0]
  ○ macrophage monocytes [1 or 0]
  ○ epithelial kidney [1 or 0]
  ○ embryo kidney [1 or 0]

- Germ layer the cells originate from:

  ○ ectoderm [1 or 0]
  ○ mesoderm [1 or 0]
  ○ endoderm [1 or 0]

- Biological nature of cell used:

  ○ cancer [1 or 0]

Nanoform characteristic:

- Minimal size - minimum size of nanoparticle in the sample, quantitative factor, derived from TEM images: 5 - 166.1 [nm]

STEP 12. New samples mapping 12.

In this step, the standardized input samples should be mapped (transformed) into principal components retaining all parameters from the L-PCA algorithm performed on the training set.

STEP 13. New samples scaling 13.

The input parameters should be standardized by scaling the original values to the mean and standard deviation values calculated for the training set, according to the equation (Eq.3):

$$z_{CX} = \frac{x_{CX} - m_X}{s_X} \qquad \text{(Eq.3)}$$

Where: zcx - standardized value of 'X feature' for the input 'C sample'; $x_{BX}$ - original value of 'X feature' for the input 'C sample'; mx - mean value of 'X feature' for all objects in the training set; sx - standard deviation of 'X feature' for all objects in the training set.

STEP 14. Predicting 14 genotoxic effects for the new sample

In this step the developed model is used to predict biological response for the new samples - new, untested nanoform(s), and defined cell line(s). As a result, a positive or negative genotoxic response is assigned to the defined samples.

## Prediction case study of method B

The presented case study is carried out on new, untested $SiO_2$ nanoforms, and defined cell line, for which the induction of the genotoxic effect is unknown. Predicting the unknown activity requires performing all 14 steps included in method B and shown in Figure 1. The algorithm can be executed by means of any programming language and/or data analysis software.

After performing steps 1-10 of method B completed with the correctly reconstructed QSAR model, confirmed by the convergence of statistical parameters for training and validation set with these presented in this document; a new nanoform and cell line (Caco-2) was defined in step 11:

Cell line characteristics:

- Cell line origin:

  ∘ human: 1
  ∘ mouse: 0
  ∘ hamster: 0
  ∘ rat: 0

- Cell type and organ of origin:

  ∘ fibroblast embryo: 0
  ∘ epithelium colon: 1
  ∘ epithelium lung: 0
  ∘ neuroblasts brain: 0
  ∘ keratinocytes skin: 0
  ∘ mononuclear cell peripheral blood (lymphocytes, monocytes): 0
  ∘ endothelium umbilical vein: 0
  ∘ lymphoblast spleen: 0
  ∘ epithelium ovary: 0
  ∘ fibroblast lung: 0
  ∘ macrophage monocytes: 0
  ∘ epithelial kidney: 0
  ∘ embryo kidney: 0

- Germ layers the cells originate from:

  ∘ ectoderm: 0
  ∘ mesoderm: 0
  ∘ endoderm: 1

- Biological nature of cell used:

  ∘ normal: 0

  ∘ cancer: 1

Nanoform characteristics:

- Minimal size - minimum size of nanoparticle in the sample, quantitative factor, derived from TEM images: 35 nm

Subsequently, the newly defined sample underwent mapping in step 12 (only cell line characteristics) and scaling in step 13 (both cell line characteristic and minimal particle size of $SiO_2$). The obtained results are as follows (Table 2):

*Table 2. Inputs of newly defined sample in Example_2 for the predictions with the QSAR model in method B*

| Sample ID | Cell line characteristic | | Nanoform characteristic |
|---|---|---|---|
| | PC1 | PC2 | Minimum particle size |
| Example_2 | 0.867768 | 1.350752 | 0.351324 |

In the last 14 step, the scaled score values of PC1, PC2 for cell line characteristics, and scaled minimum particle size of $SiO_2$, were used as inputs to make predictions with the QSAR model in method B: decision tree classifier. The application of a recreated B model resulted in: <u>negative response</u> of genotoxic effect for the newly defined sample of example.

**Claims**

1. A computational method for prediction of silica dioxide nanoparticles mutagenic effects to human health, based on QSAR modelling,
   **characterized in that** the method including:

   providing datasets of $SiO_2$ nanoforms and cell line characteristics with corresponding positive/negative responses observed in micronucleus assay, and used as a training set and a validation set;
   reduction of the multidimensionality of cell line characteristic dataset to two first principal components, PC1 and PC2, explaining more than 70% of deviation using L-PCA, pre-processing of PC1, PC2 and minimal particle size of $SiO_2$ through standardization (4), and
   reconstruction of the Support Vector Machines Classifier with inputs for the training set matrices of PC1 and PC2 describing cell lines and vector of $SiO_2$ nanoforms size, and vector of corresponding biological responses in micronucleus assay;
   a new samples mapping into PC1 and PC2 retaining all parameters from the L-PCA algorithm carried out for the training set, where the new samples are described through a value of nanoforms' minimal size within the indicated range, and a cell line characteristic through indicated adherence to the particular group with the binary scale, where the characteristics should be as follows:

   Minimal size - minimum size of nanoparticle in the sample, quantitative factor, derived from TEM images: 5 - 224.8 [nm]
   Cell line origin:

   human [1 or 0]
   Cell type and organ of origin:
   epithelium lung [1 or 0]
   fibroblast adipose tissue [1 or 0]
   mononuclear cell peripheral blood (lymphocytes, monocytes)
   epithelium colon
   endothelium umbilical vein
   Germ layer the cells originate from:

   endoderm [1 or 0]
   Biological nature of cell used:
   cancer [1 or 0];
   application of the developed QSAR model for a newly defined samples and prediction as positive or negative based on provided descriptors.

2. The computational method according to claim 1, wherein the method using the following steps:

   datasets preparation (1)
   datasets splitting (2) into a training set and a validation set,
   L-PCA (3) for the training set,
   standardization (4) for the training set,
   model calibration (5) for the training set,

internal validation (6) for the training set,
validation set mapping (7),
validation set scaling (8),
prediction (9) for the validation set,
external validation (10),
new samples defining (11)
new samples mapping (12)
new samples scaling (13)
predicting (14) mutagenic effects for the new sample

3. A computational method for prediction of silica dioxide nanoparticles genotoxic effects to human health, based on QSAR modelling,
**characterized in that** the method including:

providing datasets of $SiO_2$ nanoforms and cell line characteristics with corresponding positive/negative responses observed in comet assay, and used as a training set and a validation set;
reduction of the multidimensionality of cell line characteristic dataset to two first principal components, PC1 and PC2, explaining more than 70% of deviation using L-PCA, pre-processing of PC1, PC2 and minimal particle size of $SiO_2$ through standardization, and
reconstruction of the Decision Tree Classifier with inputs for the training set matrices of PC1 and PC2 describing cell lines and vector of $SiO_2$ nanoforms size, and vector of corresponding biological responses in comet assay;
mapping of new samples into PC1 and PC2 retaining all parameters from the L-PCA algorithm carried out for the training set, where the new samples are described through a value of nanoforms' minimal size within the indicated range, and a cell line characteristic through indicated adherence to the particular group with the binary scale, where the characteristics should be as follows:

Minimal size - minimum size of nanoparticle in the sample, quantitative factor, derived from TEM images: 5 - 166.1 [nm]
Cell line origin:

human [1 or 0]
mouse [1 or 0]
hamster [1 or 0]
rat [1 or 0]

Cell type and organ of origin:

fibroblast embryo [1 or 0]
epithelium colon [1 or 0]
epithelium lung [1 or 0]
neuroblasts brain [1 or 0]
keratinocytes skin [1 or 0]
mononuclear cell peripheral blood (lymphocytes, monocytes) [1 or 0]
endothelium umbilical vein [1 or 0]
lymphoblast spleen [1 or 0]
epithelium ovary [1 or 0]
fibroblast lung [1 or 0]
macrophage monocytes [1 or 0]
epithelial kidney [1 or 0]
embryo kidney [1 or 0]
Germ layer the cells originate from:

ectoderm [1 or 0]
mesoderm [1 or 0]
endoderm [1 or 0]
Biological nature of cell used:
cancer [1 or 0];
application of the developed QSAR model for the newly defined samples and prediction as positive or negative based on provided descriptors.

4. The computational methods according to claim 3, wherein the method using the following steps:

datasets preparation (1)
datasets splitting (2) into a training set and a validation set,
L-PCA (3) for the training set,
standardization (4) for the training set,
model calibration (5) for the training set,
internal validation (6) for the training set,
validation set mapping (7),
validation set scaling (8),
prediction (9) for the validation set,
external validation (10),
new samples defining (11)
new samples mapping (12)
new samples scaling (13)
predicting (14) genotoxic effects for the new sample.

Fig. 1.

**EUROPEAN SEARCH REPORT**

Application Number

EP 23 46 1610

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HALDER AMIT KUMAR ET AL: "A unified in silico model based on perturbation theory for assessing the genotoxicity of metal oxide nanoparticles", CHEMOSPHERE, PERGAMON PRESS, OXFORD, GB, vol. 244, 27 November 2019 (2019-11-27), XP086013370, ISSN: 0045-6535, DOI: 10.1016/J.CHEMOSPHERE.2019.125489 [retrieved on 2019-11-27] * the whole document * | 1-4 | INV. G16C60/00 |
| A | LI JING ET AL: "Nano-QSAR modeling for predicting the cytotoxicity of metallic and metal oxide nanoparticles: A review", ECOTOXICOLOGY AND ENVIRONMENTAL SAFETY, vol. 243, 1 September 2022 (2022-09-01), page 113955, XP093041761, US ISSN: 0147-6513, DOI: 10.1016/j.ecoenv.2022.113955 * the whole document * | 1-4 | |
| A | SHAYANFAR ALI ET AL: "Modeling to predict the cytotoxicity of SiO2 and TiO2 nanoparticles", JOURNAL OF RESEARCH IN PHARMACY, vol. 23, no. 2, 23 February 2019 (2019-02-23), pages 267-274, XP093110499, ISSN: 2630-6344, DOI: 10.12991/jrp.2019.133 * the whole document * | 1-4 | TECHNICAL FIELDS SEARCHED (IPC) G16C |

-/--

The present search report has been drawn up for all claims

| Place of search | Date of completion of the search | Examiner |
|---|---|---|
| The Hague | 8 December 2023 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

Europäisches
Patentamt
European
Patent Office
Office européen
des brevets

## EUROPEAN SEARCH REPORT

Application Number

EP 23 46 1610

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | "Abstracts from the Environmental Mutagenesis and Genomics Society, 45th Annual Meeting, September 13-17, 2014, Orlando, Florida", ENVIRONMENTAL AND MOLECULAR MUTAGENESIS, WILEY-LISS, INC. CHICHESTER, GB, vol. 55, 22 August 2014 (2014-08-22), pages S1-S67, XP071736858, ISSN: 0893-6692, DOI: 10.1002/EM.21895 * Abstract P15: Prediction of genotoxicity of nano metal oxides by computational metew decision tree QSAR model; page S43 * | 1-4 | |
| | ----- | | |

TECHNICAL FIELDS
SEARCHED (IPC)

The present search report has been drawn up for all claims

| Place of search | Date of completion of the search | Examiner |
|---|---|---|
| The Hague | 8 December 2023 | Nurmi, Jussi |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 16201789 A1

**Non-patent literature cited in the description**

- **RUSSELL** ; **BURCH**. *The Principles of Humane Experimental Technique*, 1959